(19) Europäisches Patentamt
European Patent Office
Office européen des brevets

(11) Veröffentlichungsnummer : **0 543 470 B1**

(12) **EUROPÄISCHE PATENTSCHRIFT**

(45) Veröffentlichungstag der Patentschrift :
**10.08.94 Patentblatt 94/32**

(51) Int. Cl.$^5$ : **C07D 317/72,** C07C 41/56, C07C 43/305

(21) Anmeldenummer : **92250289.3**

(22) Anmeldetag : **09.10.92**

(54) **Cyclische Isolongifolanon-Ketale, ihre Herstellung und ihre Verwendung.**

(30) Priorität : **19.11.91 DE 4138732**

(43) Veröffentlichungstag der Anmeldung :
**26.05.93 Patentblatt 93/21**

(45) Bekanntmachung des Hinweises auf die Patenterteilung :
**10.08.94 Patentblatt 94/32**

(84) Benannte Vertragsstaaten :
**CH DE ES FR GB LI NL**

(56) Entgegenhaltungen :
**DE-A- 2 336 798**
**GB-A- 1 505 821**
**CHEMICAL ABSTRACTS, vol. 81, no. 5, 5. August 1974, Columbus, Ohio, US;abstract no. 25442f,**
**CHEMICAL ABSTRACTS, vol. 89, no. 7, 14. August 1978, Columbus, Ohio, US;abstract no. 59977q,**

(73) Patentinhaber : **Dragoco Gerberding & Co Aktiengesellschaft**
**Dragocostrasse 1**
**D-37603 Holzminden (DE)**

(72) Erfinder : **Brunke, Ernst-Joachim, Dr.**
**Pippingsbusch 3**
**W-3450 Holzminden (DE)**
Erfinder : **Schatkowski, Dietmar**
**Weststrasse 7**
**W-3457 Stadtoldendorf (DE)**

(74) Vertreter : **Eikenberg, Kurt-Rudolf et al**
**Patentanwälte Dr. K.R. Eikenberg**
**H.D. Brümmerstedt und Uwe Thömen**
**Schackstrasse 1**
**D-30175 Hannover (DE)**

**EP 0 543 470 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Heutzutage bestehen industriell erzeugte Parfümöle zu einem großen Teil aus synthetischen Riechstoffen. Die traditionelle Verwendung von etherischen Ölen oder Extrakten pflanzlichen und tierischen Ursprungs ist immer noch wichtig auf dem Gebiet der alkoholischen Parfümerie. Die Parfümierung von Waschmitteln, Seifen, Haushaltsreinigern und ähnlichen Produkten stellt spezielle technische Probleme bezüglich der Stabilität der Riechstoffe bzw. der Haftfestigkeit. Solche Anforderungen können nur durch maßgeschneiderte Riechstoffe gelöst werden. Dementsprechend werden Parfümöle für technische Verbrauchsgüter im wesentlichen aus synthetischen Riechstoffen komponiert. Da derartige Parfümöle in großen Mengen benötigt werden, haben sich in den letzten Jahrzehnten alle bedeutenden Parfümhäuser bzw. Riechstoffhersteller mit der Erforschung entsprechender Riechstoffe beschäftigt.

Seit etwa 10 Jahren ist mehr und mehr festzustellen, daß synthetische Riechstoffe, die aufgrund des niedrigen Preises und der hohen Stabilität für die technische Parfümerie geplant waren, und die entsprechend auch zunächst im Markt positioniert waren, inzwischen zunehmend Eingang in die Feinparfümerie gefunden haben. Parfümeure nutzen das parfümistische Know-how, das sie mit synthetischen Riechstoffen in ihren Arbeiten für technische Parfümierungen gewonnen haben, auch für die Feinparfümerie, wenn entsprechende ästhetische Möglichkeiten vorliegen. Ein erfolgreicher neuer Riechstoff hat heutzutage folgenden Ansprüchen zu genügen: er sei

1. von olfaktorisch-ästhetisch hohem Wert und somit universell einsetzbar;
2. stabil in den meisten technischen Anwendungen;
3. preiswert bezüglich seines Preis-/Leistungsvermögens;
4. basierend auf allgemein verfügbaren Rohstoffen, möglichst nachwachsenden Rohstoffen.

Ein solcher in großen Mengen verfügbarer Rohstoff natürlichen Ursprungs ist Longifolen (**1**), das als Hauptkomponente im indischen Terpentinöl und als Nebenkomponente in zahlreichen anderen Terpentinöl-Sorten sowie weiteren ätherischen Ölen vorkommt.

Vor Ca. 20 Jahren sind von Forschungslaboratorien der Riechstoffindustrie eine Reihe von Folgeprodukten des Longifolens (**1**) hergestellt worden, die über Riechstoffeigenschaften verfügen. Wie G. Ohloff in seinem Buch "Riechstoffe und Geruchssinn" (Springer-Verlag, Berlin, 1990, ISBN-Nr. 3-540-52560-2, Seiten 87-88) zusammenfassend berichtet, stammen vom Longifolen (**1**) mindestens 4 kommerzielle Riechstoffe ab, während von dem durch Isomerisierungsreaktion aus Longifolen (**1**) zugänglichen Isolongifolen (**2**) nachweislich 13 Produkte gehandelt werden.

Chemie und Geruchseigenschaften von Derivaten des Isolongifolens (**2**) werden von G. Färber und H. Tan zusammenfassend beschrieben:
"Riechstoffe aus Isolongifolen", G. Färber, Parfümerie & Kosmetik, 68, 18 (1987)
"Der Gebrauch von Riechstoffen aus Isolongifolenen in der Parfümerie", H. Tan, Parfümerie & Kosmetik, 67, 564 (1986)
Die aus Isolongifolen (**2**) durch Epoxidation, Prins-Reaktion (Umsetzen mit Formaldehyd) oder Allyloxidation zugänglichen Derivate gelten als die im Vergleich zu den Longifolen-Derivaten sensorisch wertvolleren Riechstoffe von warm-holzigem Geruchstyp, z. T. mit etwas Ambra-artigem Effekt (Ohloff loc. cit.).

Das durch Umsetzung von Isolongifolen (**2**) mit Persäuren zugängliche Epoxyd (**3**) läßt sich in bekannter Weise in Gemische der epimeren Ketone **4a/4b** überführen, wobei diese Ketongemische in Abhängigkeit von den Produktionsbedingungen unterschiedliche Isomerenverteilungen und Geruchseffekte aufweisen. Das Keton **4a** entsteht bevorzugt unter kinetischer Reaktionskontrolle, während das Keton **4b** das thermodynamisch stabilere Epimere ist.

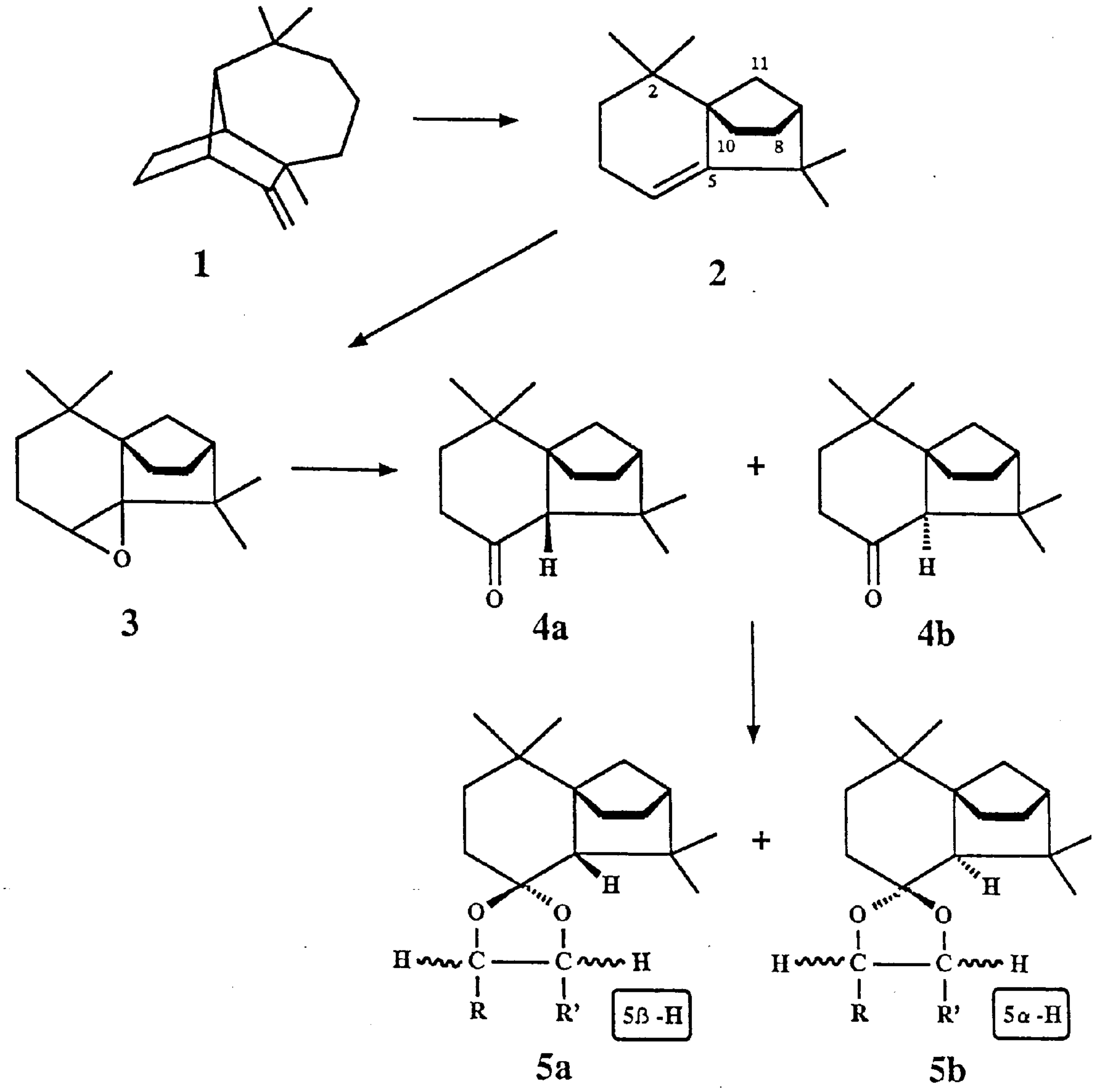

1

2

3

4a + 4b

5a + 5b

| | | | |
|---|---|---|---|
| 5a | R=H | R'=H | 5b |
| 6a | R=H | R'=Me | 6b |
| 6c | R=Me | R'=H | 6d |
| 7a | R=H | R'=Et | 7b |
| 7c | R=Et | R'=H | 7d |
| 8a, 8c | R=Me (α, ß) | R'=Me (α, ß) | 8b, 8d |

Entsprechend dem beschriebenen Stand der Technik gilt dieses Gebiet der Riechstoffchemie als besonders gut untersucht. Neben einigen Longifolen- und Isolongifolen-Derivaten mit Riechstoff-Eigenschaften sind eine größere Zahl von solchen Derivaten ohne bzw. ohne besonderen olfaktorischen Wert bekannt. Es ist daher besonders überraschend, daß auf dem Gebiet der Longifolen-Derivate nunmehr neue, wertvolle Riechstoffe gefunden werden konnten, wie dies die hier beanspruchten neuen cyclischen Acetale der allgemeinen Formel **A** sind. Die Acetale der allgemeinen Formel **A** verfügen über ganz eigenständige olfaktorische Eigenschaften, die sich deutlich von den bekannten Riechstoffen aus Isolongifolen (**2**) abheben und diese auch übertreffen. Die neuen Verbindungen der allgemeinen Formel **A** besitzen stark holzige Geruchseigenschaften mit blumigfrischen Effekten und samtigem Moos-/Ambra-Charakter (siehe Beispiel 1); sie sind besonders langhaftend und fixierend.

Zur Herstellung der Verbindungen der allgemeinen Formel **A** wurde Longifolen (**1**) in bekannter Weise durch Behandeln mit einem Gemisch aus Essigsäure und Schwefelsäure [U.R. Nayak, S. Dev, Tetrahedron 8, 42-48 (1960)] oder mit Bortrifluorid-Etherat [R.E. Beyler, G. Ourisson, J.Org.Chem. 30, 2838-2839 (1965)] zu Isolongifolen (**2**) isomerisiert. Das aus Isolongifolen (**2**) durch Epoxydation erhaltene Epoxid (**3**) [L.K. Lala, J.B. Hall, J.Org.Chem. 35, 1172, (1970); J.R. Prahlad, R. Ranganathan, W. Ramdas Nayak, T.S. Santhanakrishnan, S. Dev, Tetrahedron Lett. 8, 417 (1964)] wurde in bekannter Weise in das Gemisch der epimeren Ketone **4a/4b** umgewandelt [R. Ranganathan, U.R. Nayak, T.S. Santhanakrishnan, S. Dev, Tetrahedron 26, 621 (1970)]. Bei der Epoxyd-Ringöffnung entstand unter den bekannten Reaktionsbedingungen (kinetische Kontrolle) ein an **4a** angereichertes Ketongemisch **4a/4b** (siehe Beispiel 2: **4a**:**4b** = 96:4). Es ist bekannt, daß **4a** unter dem Einfluß basischer Katalysatoren oder durch thermische Belastung in das thermodynamisch stabilere Keton **4b** isomerisiert wird. In Abhängigkeit von den Reaktionsbedingungen werden Gleichgewichtsgemische aus **4a/4b** erhalten [L.K. Lala, J. Org. Chem. 36, 2560-2561 (1971)].

In unserem Beispiel 3 zeigen wir eine bisher nicht beschriebene Isomerisierung, die von einem Ketongemisch **4a/4b** (96:4) zu einem an **4b** besonders hoch angereicherten Gleichgewichtsgemisch **4a/4b** (9:91) führte. Die Ketone **4a/4b** sind durch destillative bzw. chromatographische Methoden auch in reiner Form isoliert und spektroskopisch charakterisiert worden [C.W. Greengrass, R. Ramage, Tetrahedron 31, 689-694 (1975)].

Die neuen cyclischen Acetale der Formel **A** werden aus den Ketonen **4a**, **4b**, die in reiner bzw. angereicherter Form oder als Gleichgewichtsgemische vorlagen, in an sich bekannter Weise durch Umsetzen mit aliphatischen 1,2-Diolen unter Säurekatalyse und Wasserabscheidung dargestellt. Die Wasserabscheidung wurde bevorzugt unter Verwendung von als Schleppmittel geeigneten inerten Lösungsmitteln bei Siedetemperatur ausgeführt (Beispiele 5 - 10).

Bei Verwendung verschiedener Lösungsmittel, beispielsweise Toluol, Cyclohexan, Benzin-Fraktionen oder n-Pentan entstehen die epimeren Ketale in unterschiedlichem Verhältnis (Beispiel 5). So wurden aus **4a/4b** (86:14, aus Beispiel 2) bei Einwirkung von Ethylenglykol in Toluol die Ketale **5a/5b** im Verhältnis 3:2 erhalten. Bei Verwendung von n-Pentan wurde selbst bei deutlich verlängerter Reaktionszeit ein Ketalgemisch **5a/5b** im Verhaltnis 99:1 erhalten. Die Ketale der Formel **A** wurden wahlweise durch Destillation von gegebenenfalls noch vorhandenen Edukt-Keton **4a/4b** getrennt.

Das durch Basen-Katalyse equilibrierte Ketongemisch **4a/4b** (9:91, aus Beispiel 3) wurde ebenfalls Ketalisierungsreaktionen unterzogen. Hierbei wurden ebenfalls in Abhängigkeit vom verwendeten Lösungsmittel unterschiedlich zusammengesetzte Ketalgemische **5a/5b** erhalten, die sich jedoch von den aus dem Ketongemisch **4a/4b** (86:14, aus Beispiel 2) erhaltenen Ketalgemischen **5a/5b** bezüglich ihrer relativen Konzentrationen unterscheiden. Dieses Reaktionsverhalten kann durch kinetisch kontrollierte Reaktionsabläufe bei der Ketalisierung verstanden werden.

In Analogie zu den Ketalen **5a/5b** wurden durch Umsetzen von **4a/4b** mit 1,2-Propandiol die Ketale **6a/6b/6c/6d**, mit 1,2-Butandiol die Ketale **7a/7b/7c/7d**, mit 2,3-Butandiol die Ketale **8a/8b/8c/8d** jeweils als Gemisch erhalten. Die Methyl- oder Ethylgruppen des Ketalrestes können dabei α- oder β-konfiguriert sein.

Es werden die Verbindungen **5a, 6a, 6c, 7a, 7c, 8a, 8c** als 5β-konfiguriert und die Verbindungen **5b, 6b, 6d, 7b, 7d, 8b, 8d** als 5α-konfiguriert festgelegt. Da ausgehend von (+)-Longifolen über die chiralen Ketone **4a/4b** die ebenfalls chiralen Ketale der allgemeinen Formel **A** erhalten wurden, liegen diese als Gemisch von Epimeren (**5a/5b**), bzw. Diastereomeren vor; **6a/6b** und **7a/7b** sind konstitutionsisomer zu **6c/6d** bzw. **7c,d**. Ausgehend von (-)-Longifolen sind jeweils die enantiomeren Verbindungen **5a,b - 8a-d** zugänglich.

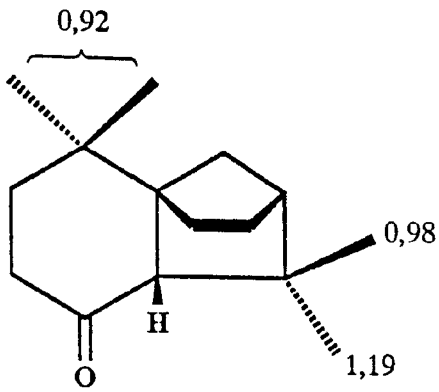


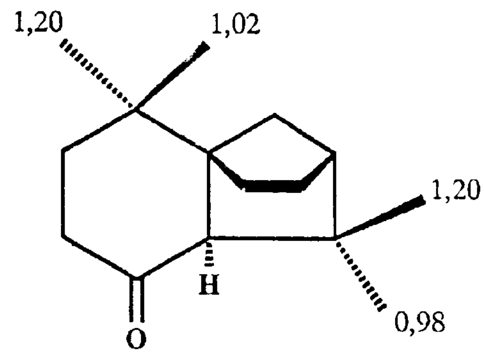


**4a** **4b**

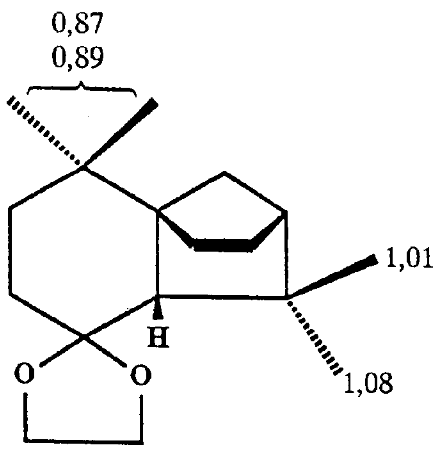


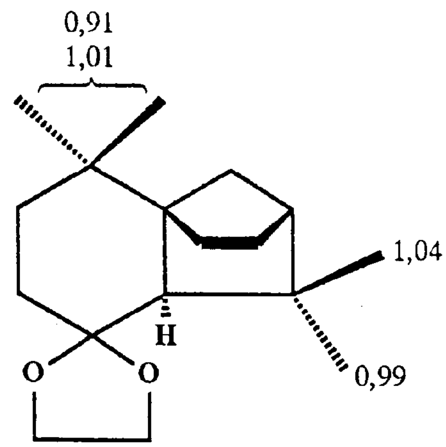


**5a** **5b**

## 1H-NMR-Werte, δ [ppm]

Da die Zusammensetzung der Ketalgemische von den Reaktionsbedingungen abhängt (Beispiel 5) und die einzelnen Diastereomeren in reiner Form gewonnen werden können (Beispiele 6, 7), sind beliebige Mischungsverhältnisse einstellbar.

Die Strukturzuordnung der neuen Verbindungen **5 - 8** wurde aufgrund der spektroskopischen Meßergebnisse getroffen (Beispiele 5- 10). So wurden die $^1$H-NMR-Spektren der Verbindungen **5a**, **5b** (Abb. 1, 2) in Analogie zu den von C.W. Greengrass und R. Ramage, Tetrahedron 31, 689 (1975) für die Ketone **4a**, **4b** gegebenen Zuordnungen interpretiert.

Die neuen Verbindungen der Formel **A** sind aufgrund ihrer Geruchseigenschaften und ihrer Stabilität als Riechstoffe sehr gut geeignet. Sie können in Parfüm-Kompositionen beliebigen Geruchstyps sowohl als eine der Hauptkomponenten als auch im Spurenbereich vorteilhaft eingearbeitet werden. Die gegebenen Beispiele sind daher nicht einschränkend zu verstehen.

BEISPIEL 1

Herstellung von Isolongifolen (**2**)

Zu einer auf 60 °C erhitzten Lösung aus 90 g Toluol und 10 g (0,07 mol) $BF_3$-Etherat wurden innerhalb von 30 min 204 g (0,79 mol) Longifolen (**1**) (80 % ex Terpentinöl indisch $[\alpha]_D$ = + 39,4°) zugetropft, 3 h bei 100 °C nachgerührt, danach auf Raumtemperatur heruntergekühlt und mit Sodalösung und Wasser neutralgewaschen. Nach Trocknung über $Na_2SO_4$ wurde das Lösungsmittel unter vermindertem Druck abdestilliert. Es verblieben 198 g (70,2 %ig nach GC) Rohprodukt.
Gaschromatogramm (HP 5890, DBWAX-30 N, 30 m, 150 °C - 240 °C, 8 °C/min).

BEISPIEL 2

Herstellung von Isolongifolanon-Gemisch **4a/4b** (86:14)

In einem Dreihalskolben mit Rückflußkühler und Tropftrichter wurden 198 g (0,68 mol) Isolongifolen (**2**) (70,2 %ig nach GC) aus Beispiel 1 80 g Toluol, 68 g (1,48 mol) Ameisensäure vorgelegt und auf 60 - 70 °C erhitzt. Anschließend wurden über einen Zeitraum von 1 Std. 136 g (1,4 mol) $H_2O_2$ (35 %ig) zugetropft. Nach 3 Std. Rühren bei 80 - 85 °C wurde auf Raumtemperatur heruntergekühlt und aufgearbeitet. Die abgetrennte organische Phase wurde mit Sodalösung und Wasser neutralgewaschen, über $Na_2SO_4$ getrocknet und das Lösungsmittel unter reduziertem Druck abdestilliert. Es verblieben 199 g Rohprodukt. GC **4a** (71 %); **4b** (2,5 %) [96 : 4].

Eine Destillation über eine 15 cm Vigreux-Kolonne ergab 165 g rohes **4a/4b**; $Kp_{2mm}$ 120 - 153 °C. GC: **4a** (74 %), **4b** (3 %).

Anschließende Feindestillation über eine 50 cm Metallfüllkörperkolonne ergab: 110 g **4a/4b** (74,4 % d. Th.) $Kp_{2mm}$ 137 - 141 °C; GC **4a** (77 %), **4b** (12,8 %) [86 : 14]
D 20/4 = 1,0037
n 20/D = 1,5006
[α] 20/D = - 8,5°
Gaschromatogramm: Bedingungen siehe Beispiel 1
GC/MS: HP 5970 DBWAX-60 N, 60 m 60 - 240 °C, 4°/min

**4a** *Rt = 40,47′*
MS: m/e (%) = 220 (100, $M^+$ ), 205 (41), 191 (82), 177 (41), 164 (62), 149 (50), 121 (56), 107 (40), 83 (27), 55 (21), 41 (17).

**4b** *Rt = 41,41′*
MS: m/e (%) = 220 (69, $M^+$ ), 205 (23), 191 (55), 177 (44), 164 (100), 149 (55), 121 (53), 107 (37), 91 (21), 55 (21), 41 (19).

BEISPIEL 3

C-3-Epimerisierung von Isolongifolanon (**4a/4b**)

In einem 4 l Dreihalskolben wurden 880 g (4 mol) rohes (undestilliertes) Ketongemisch **4a/4b** aus Beispiel 2; (Reinheit nach GC: **4a** (63,3 %), **4b** (2,48 %) [96 : 4]); 750 g Methanol, 40 g (0,5 mol) NaOH 50 %ig vorgelegt und insgesamt 8 Std. unter Rückfluß gerührt. Nach dieser Zeit wurde mit 30 g (0,5 mol) Eisessig versetzt und auf Raumtemperatur abgekühlt. Nach dem Abdestillieren des Lösungsmittels unter vermindertem Druck wurde der Rückstand in Wasser aufgenommen. Die organische Phase wurde abgetrennt. Die wässerige Phase wurde mit 100 ml Benzin extrahiert. Die vereinigten organischen Phasen wurden mit Sodalösung und danach mit Wasser neutralgewaschen, über $Na_2SO_4$ getrocknet und vom Lösungsmittel unter vermindertem Druck befreit. Es verblieben 860 g dunkelbraunes Öl.
GC: **4a** (6,3 %), **4b** (59,3 %) [9 : 91]

BEISPIEL 4

C-3-Epimerisierung von reinem Isolongifolanon (**4a/4b**)

In einem 2 l Dreihalskolben wurden 440 g (2 mol) feindestilliertes Ketongemisch **4a/4b** aus Beispiel 2; (Reinheit nach GC: **4a** (77 %), **4b** (12,8 %) [86 : 14], 400 ml Methanol, 20 g (0,25 mol) NaOH 50%ig insgesamt 5 Std. unter Rückfluß gerührt. Nach dem Abkühlen auf Raumtemperatur versetzte man mit 15,5 g (0,25 mol) Eisessig und destillierte das Lösungsmittel unter vermindertem Druck ab.

Der verbliebene Rückstand wurde in Wasser aufgenommen. Die organische Phase wurde abgetrennt und die wässerige Phase einmal mit 100 ml Benzin extrahiert. Die vereinigten organischen Phasen wurden mit Sodalösung und Wasser neutralgewaschen und eingeengt. Es verblieben 430 g braunes Öl.
GC: **4a** (9,2 %), **4b** (79,5 %) [1 : 9]

Destillation über eine 15 cm Vigreux-Kolonne ergab 411 g **4a/4b** ($Kp_{2mm}$ 135 - 157 °C). Anschließende Feindestillation über eine 40 cm Metallfüllkörperkolonne ergab 399 g (90,7 % d.Th.) **4a/4b** $Kp_{2mm}$ 139 - 142 °C.
GC: **4a** (13,4 %), **4b** (80,6 %) [14 : 86]
D 20/4 = 1,0042
n 20/D = 1,5007
[α] 20/D = - 34,7°

BEISPIEL 5

Umsetzung von Isolongifolanon **4a/4b** mit Ethylenglycol

In einem 1 l Dreihalskolben mit Wasserabscheider wurden jeweils 220 g (0,6 mol) Ketongemisch **4a/4b** aus Beispiel 2, 3 oder 4, 186 g (3 mol) Ethylenglycol, 1 g p-Toluolsulfonsäure sowie 300 ml Lösungsmittel (Toluol, Cyclohexan, Benzin (63-80°C), n-Pentan) vorgelegt und 48 - 78 h bei Siedetemp. und unter Wasserabscheidung gerührt. Im Verlauf der Reaktion wurden jeweils ca. 20 ml Wasser abgeschieden.

Nach dem Abkühlen auf Raumtemperatur wurde mit Sodalösung und Wasser neutralgewaschen, über $Na_2SO_4$ getrocknet und vom Lösungsmittel unter vermindertem Druck befreit. Man erzielt jeweils 245 - 265 g Rohprodukt als gelbes bzw. braunes Öl.

Destillation über eine 15 cm Vigreux-Kolonne ergab jeweils ca. 230 g rohes **4a/4b** ($Kp_{2mm}$ 68 - 170 °C). Die anschließende Feindestillation über eine 40 cm Metallfüllkörperkolonne ergab jeweils ca. 140 g (53 % d. Th.) **5a/5b** als hellgelbes Öl.

Die Ergebnisse sind in Tabelle 1 zusammengefaßt.

Tabelle 1 Produkte der Umsetzung von **4a/4b** mit Ethylenglycol/p-Toluolsulfonsäure

| | Edukte (GC-%) 4a | + 4b | [4a/4b] | Lösungsmittel | Reaktions- -Temp. | -Zeit | Produkt-Zusammensetzung (GC-%) 4a | 4b | 5a | 5b | [5a/5b] |
|---|---|---|---|---|---|---|---|---|---|---|---|
| a) | 71 % | 2.5 % | [96: 4] | Benzin (63-80°C) | 70-72°C | 70 h | 7.2 | 3.8 | 53.6 | 2.2 | [96: 4] |
| | 71 % | 2.5 % | [96: 4] | Cyclohexan | 90-92°C | 48 h | 5.1 | 4.8 | 51.3 | 3.8 | [93: 7] |
| | 71 % | 2.5 % | [96: 4] | Toluol | 120 °C | 24 h | 1.2 | 10.4 | 37.8 | 19.6 | [66:34] |
| | (Rohprodukte aus Beispiel 2) | | | | | | | | | | |
| b) | 77 % | 12.8 % | [86:14] | n-Pentan | 46-48°C | 84 h | 17.1 | 11.1 | 60.1 | 0.8 | [99: 1] |
| | 77 % | 12.8 % | [86:14] | Benzin (63-80°C) | 70-72°C | 72 h | 1.8 | 14.6 | 57.9 | 14.3 | [80:20] |
| | 77 % | 12.8 % | [86:14] | Cyclohexan | 90-92°C | 25 h | 2.4 | 10.0 | 65.5 | 11.3 | [85:15] |
| | 77 % | 12.8 % | [86:14] | Toluol | 120 °C | 25 h | - | - | 84.6 | 14.3 | [86:14] |
| | (Destillat aus Beispiel 2) | | | | | | | | | | |
| c) | 6.3 % | 59.3 % | [ 9:91] | Benzin (63-80°C) | 70-72°C | 78 h | 1.2 | 14.5 | 28.6 | 19.8 | [59:41] |
| | 6.3 % | 59.3 % | [ 9:91] | Cyclohexan | 90-92°C | 78 h | 2.1 | 11.5 | 36.9 | 13.6 | [73:27] |
| | 6.3 % | 59.3 % | [ 9:91] | Toluol | 120 °C | 78 h | 2.9 | 8.4 | 37.7 | 13.3 | [73:27] |
| | (aus Beispiel 3) | | | | | | | | | | |
| d) | 13.4 % | 80.6 % | [14:86] | Benzin (63-80°C) | 70-72°C | 72 h | - | 22.9 | 35.6 | 35.1 | [50:50] |
| | 13.4 % | 80.6 % | [14:86] | Toluol | 120 °C | 48 h | 1.5 | 3.3 | 67.9 | 19.8 | [77:23] |
| | (aus Beispiel 4) | | | | | | | | | | |

BEISPIEL 6

Gewinnung von 5-Ethylendioxi-3β-H-isolongifolan (**5a**)

20 g feindestilliertes Ketalgemisch aus Beispiel 5b; (Reinheit nach CC: **5a** (84,6 %), **5b** (14,3 %) [86:14], wurden an einer 1 m Drehbandkolonne nochmals feindestilliert. Dabei wurden 2,8 g **5a** als hellgelbes Öl erhalten, $Kp_{2mm}$ 142 - 143 °C.

GC: **5a** (98 %), **5b** (0,8 ) [99:1]

D 20/4 = 1,0510

n 20/D = 1,5051

GC/MS: Bedingungen siehe Beispiel 2

**5a** *Rt 42.94'*

MS: m/e (%) = 264 (23, $M^+$ ), 249 (9), 235 (19), 195 (20), 165 (23), 127 (42), 99 (100), 55 (10).

$^1$H-NMR: siehe Abb. 1

$^{13}$C-NMR ($CDCl_3$), Varian VXR-300): δ [ppm] = 21.86, 23.39, 26.79, 33.89 ($\underline{C}H_3$), 21.06, 25.75, 32.76, 35.89, 37.95, 61.84, 63.71 ($\underline{C}H_2$), 48.94, 52.48 ($\underline{C}H$), 33.21, 37.67, 56.42, 112.23 ($\underline{C}$).

BEISPIEL 7

Isolierung von 5-Ethylendioxi-3α-H-isolongifolan (**5b**)

1 g rohes Ketalgemisch aus Beispiel 5d (Reinheit nach GC: **5a** (35,6 %), **5b** (35,1 % [1:1]), wurden durch mehrfache (3 x) Flash-Chromatographie gereinigt.

Chromatographiebedingungen:

150 g Kieselgel 60, Korngröße 0,04 - 0,063 mm, (Fa. Merck, Art.-Nr. 9385).

Laufmittel: Benzin / Essigester = 95 / 5

Einwaage: 1 g

Ausbeute: 78 mg; GC: **5a** (3 %), **5b** (90 %) [3:97]

GC/MS: Bedingungen siehe Beispiel 2

**5b** *Rt 44,67'*

MS: m/e (%) = 264 (9, $M^+$ ), 249 (1), 235 (1), 221 (2), 191 (1), 149 (2), 127 (9), 99 (100), 55 (6), 41 (3).

$^1$H-NMR: siehe Abb. 2

$^{13}$C-NMR ($CDCl_3$), Varian VXR-300): δ [ppm] = 25.51, 26.72, 26.93, 31.09 ($\underline{C}H_3$), 26.01, 30.41, 31.65, 36.90, 37.56, 61.38, 63.05 ($\underline{C}H_2$), 49.53, 56.89 ($\underline{C}H$), 32.46, 40.99, 56.63, 111.78 ($\underline{C}$).

BEISPIEL 8

Herstellung von 5-(1'-Methylethylendioxi)-isolongifolan **6a/6b/6c/6d**

In einem 4 l-Dreihalskolben mit Wasserabscheider wurden 440 g (2 mol) feindestilliertes Ketongemisch **4a/4b** aus Beispiel 2 (Reinheit nach GC: **4a** (77 %), **4b** (12,8 %) [86 : 14], 760 g (10 mol) Propylenglycol-1.2, 600 ml Toluol, 2 g p-Toluolsulfonsäure vorgelegt und 30 Std. unter Rückfluß gerührt. Nach dem Abkühlen auf Raumtemperatur wurde mit Sodalösung und Wasser neutralgewaschen, über $Na_2SO_4$ getrocknet und das Lösungsmittel unter vermindertem Druck abdestilliert. Es verblieben 540 g hellbraunes Rohprodukt.

Eine Destillation über eine 15 cm Vigreux-Kolonne ergab 298 g (53,6 % d. Th.) **6a/6b/6c/6d**; $Kp_{2mm}$ 158 - 162 °C .

GC/MS: Bedingungen siehe Beispiel 2

*Rt 41.11'*

MS: m/e (%) = 278 (21, M+), 263 (19), 249 (40), 179 (41), 141 (39), 113 (100), 83 (20), 55 (31).

*Rt 42,03'*

MS: m/e (%) = 278 (29, $M^+$ ), 263 (22), 249 (55), 209 (40), 179 (49), 141 (42), 113 (100), 83 (21), 55 (31).

*Rt 42,11'*

MS: m/e (%) = 278 (23, $M^+$ ), 263 (21), 249 (44), 209 (35), 179 (47), 141 (39), 113 (100), 83 (21), 55 (34).

*Rt 42,47'*

MS: m/e (%) = 278 (29, $M^+$ ), 263 (21), 249 (51), 209 (37), 179 (47), 141 (44), 113 (100), 83 (21), 55 (26).

BEISPIEL 9

Herstellung von 5-(1'-Ethyl-ethylendioxi)-isolongifolan **7a/7b/7c/7d**

In einem 1 l Dreihalskolben mit Wasserabscheider wurden 220 g (1 mol) feindestilliertes Ketongemisch **4a/4b** aus Beispiel 2 (Reinheit nach GC: **4a** (77 %), **4b** (12,8 %) [86 : 14]), 270 g (3 mol) 1,2-Butandiol, 300 ml

Cyclohexan, 1 g p-Toluolsulfonsäure insgesamt 50 Std. unter Rückfluß gerührt. Nach dem Herunterkühlen auf Raumtemperatur wurde mit Sodalösung und Wasser neutralgewaschen, über $Na_2SO_4$ getrocknet. Nach Abdestillieren des Lösungsmittels unter vermindertem Druck verblieben 328 g hellbraunes Öl.

Eine anschließende Destillation an einer 15 cm Vigreux-Kolonne ergab 195 g (66,8 % d. Th.) **7a/7b/7c/7d**; $Kp_{2mm}$ 152 - 157 °C als hellgelbes Öl.

GC/MS: Bedingungen siehe Beispiel 2

*Rt 42,55'*
MS: m/e (%) = 292 (16, $M^+$), 277 (15), 263 (37), 223 (27), 193 (28),155 (26), 127 (100), 83 (13), 55 (43).

*Rt 43,37'*
MS: m/e (%) = 292 (32, $M^+$), 277 (25), 263 (56), 223 (48), 193 (41), 155 (37), 127 (100), 83 (17), 55 (49).

*Rt 43,66'*
MS: m/e (%) = 292 (19, $M^+$), 277 (19), 263 (42), 223 (45), 193 (34), 155 (28), 127 (100), 83 (13), 55 (50).

*Rt 44,29'*
MS: m/e (%) = 292 (32, $M^+$), 277 (24), 263 (57), 223 (43), 193 (41), 155 (43), 127 (100), 83 (17), 55 (50).

BEISPIEL 10

Herstellung von 5-(1′,2′-Dimethyl-ethylendioxi)-isolongifolan **8a/8b/8c/8d**

In einem 1 l Dreihalskolben mit Wasserabscheider wurden 220 g (1 mol) feindestilliertes Ketongemisch **4a/4b** aus Beispiel 2; Reinheit nach GC **4a** = 77 %; **4b** = 12,8 % (86 : 14); 270 g (3 mol) 2,3-Butandiol, 300 ml Cyclohexan, 1 g p-Toluolsulfonsäure insgesamt 50 Std. unter Rückfluß gerührt. Nach dem Herunterkühlen auf Raumtemperatur wurde mit Sodalösung und Wasser neutralgewaschen, über $Na_2SO_4$ getrocknet und das Lösungsmittel unter vermindertem Druck abdestilliert. Es verblieben 275 g hellbraunes Rohprodukt.

Eine Destillation über eine 15 cm Vigreux-Kolonne ergab 211 g (72,2 % d. Th.) **8a/8b/8c/8d** $Kp_{2.5mm}$ 158 - 162 °C als hellgelbes Öl.

D 20/4 = 1,0115
n 20/D = 1,4975
GC/MS: Bedingungen siehe Beispiel 2

*Rt 39,69'*
MS: m/e (%) = 292 (41, $M^+$), 277 (25), 263 (57), 223 (50), 193 (49), 155 (50), 127 (100), 83 (20), 55 (29).

*Rt 40,14'*
MS: m/e (%) = 292 (47, $M^+$), 277 (30), 263 (63), 223 (59), 193 (53), 155 (51), 127 (100), 83 (20), 55 (35).

*Rt 42,76'*
MS: m/e (%) = 292 (16, $M^+$), 277 (14), 263 (28), 223 (28), 193 (26), 155 (25), 127 (100), 83 (15), 55 (28).

*Rt 43,36'*
MS: m/e (%) = 292 (56, $M^+$), 277 (40), 263 (91), 223 (78), 193 (66), 155 (64), 127 (100), 83 (38), 55 (41).

BEISPIEL 11

Geruchsbeschreibungen der Ketale **5** bis **8**

Die Geruchsbewertung wurde von einem Expertengremium anhand 10 %iger ethanolischer Lösung unter Verwendung von Riechstreifen vorgenommen.

**5a/5b** [86 : 14] aus Beispiel 5b:
stark, süß-holzig, mit samtigem Ambra-Charakter und blumigen Aspekten.

**5a** aus Beispiel 6:
stark holzig mit moosigem Ambra-Charakter und frischem Effekt.

**5b** aus Beispiel 7:
holzig, hell-blumig, mit weich-erdiger Ambra-Note, etwas schwächer als Verbindung **5a**.

**6a/6b/6c/6d** (aus Beispiel 8):
stark holzig, pudrig, mit frischem Ambra-Effekt.

**7a/7b/7c/7d** (aus Beispiel 9):
trocken, holzig

**8a/8b/8c/8d** (aus Beispiel 10):
stark holzig, mit moosigen, erdigen sowie süß-animalischen Aspekten.

Alle Verbindungen verfügen über einen starken Nachgeruch, der mehrere Wochen anhält.

BEISPIEL 12

Parfümbase blumig-holzigen Typs

| | |
|---|---|
| Bergamotte Öl | 7,5 |
| Linalool | 4,0 |
| Phenylethylalkohol | 5,0 |
| Benzylacetat | 2,0 |
| Citronellol | 2,0 |
| Hedione(R) (a) | 10,0 |
| LyralR (b) | 4,0 |
| Hydroxycitronellal | 2,5 |
| Rosenoxid 1 (c) 10 % in DPG | 2,5 |
| Hexylzimtaldehyd, alpha | 7,5 |
| Patchouli Öl indonesisch | 4,0 |
| Iso-E-Super(R) (b) | 2,0 |
| Vetiverylacetat | 2,0 |
| Brahmanol F (c) | 2,0 |
| Benzylsalicylat | 2,0 |
| Hexenylsalicylat-cis 3 | 1,0 |
| Cedramber(R) (b) | 1,0 |
| Moschus Xylol | 1,0 |
| Indol 10 % in DPG | 0,5 |
| Opoponax Extrakt | 0,5 |
| Eichenmossextrakt 50 % in DPG | 5,0 |
| | 68,0 |

(a) Firmenich

(b) IFF

(c) DRAGOCO

Die Parfümbase der angegebenen Formel besitzt einen ausgewogenen blumig-holzigen Duftcharakter, der durch Zugabe von 32 Teilen **5a/5b** (80:20) deutlich verstärkt und harmonisiert wird.

BEISPIEL 13

Parfümbase vom Fougere-Typ

| | |
|---|---|
| Bergamotte Öl | 18,0 |
| Lavandinöl Super | 15,0 |
| Lilial(R) | 10,0 |
| Anisaldehyde | 3,0 |
| Coumarin | 5,0 |
| Hexylzimtaldehyd, alpha | 20,0 |
| Ambrinolepoxid 10 % in DPG | 0,5 |
| Ambroxan(R) (d) 10 % in DPG | 1,0 |
| Romaryl (c) | 10,0 |
| Pfefferminzöl | 1,0 |
| | 82,5 |

(c) DRAGOCO
(d) Henkel

Die Parfümbase der angegebenen Formel besitzt einen frisch-krautigen Fougere-Duft. Die Zugabe von 7,5 Teilen **6a/6b/6c/6d** ergibt eine Abrundung unter gleichzeitiger Betonung der ambriert holzigen Note. Die alternative Zugabe von 7,5 Teilen **8a/8b/8c/8d** ergibt ebenfalls eine sehr gewünschte Abrundung unter gleichzeitiger Betonung von animalisch-holzigen Aspekten.

BEISPIEL 14

Akkord vom grün-blumigen Typ

| | |
|---|---|
| Galbanum Öl ED | 0,5 |
| Eugenol | 1,0 |
| Methylionon-gamma | 5,0 |
| Hexenylsalicylat-cis 3 | 6,0 |
| Benzylacetat | 8,0 |
| Lignofix(R) (c) | 5,0 |
| Hedione(R) (a) | 10,0 |
| Benzylsalicylat | 10,0 |
| Hexylzimtaldehyd, alpha | 12,0 |
| Phenylethylalkohol | 15,0 |
| | 72,5 |

(a) Firmenich
(c) DRAGOCO

Das Parfümöl der angegebenen Formel besitzt einen harmonischen, blumig-grünen Duftcharakter. Durch Zugabe von 7,5 Teilen **5a/5b** bzw. alternativ hierzu **7a/7b/7c/7d** resultiert in einer sehr gewünschten Abrundung bei gleichzeitig sehr natürlich wirkendem Geruchscharakter.

## Patentansprüche

1. Cyclische Isolongifolanon-Ketale der allgemeinen Formel **A**, wobei geschlängelte Linien α- und β-Konfiguration, und R,R′ jeweils Wasserstoff-, Methyl- oder Ethylreste bedeuten,

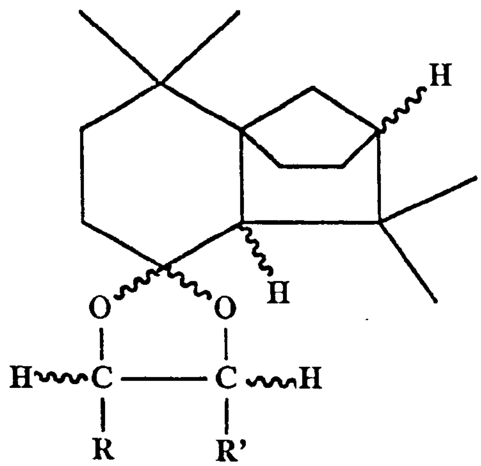

**A**

wobei die folgenden Kombinationen bevorzugt sind:
R=R′ =H
R=H, R′=Me/R=Me, R′=H
R=H, R′=Et/R=Et, R′=H
R=R′ =Me(α,β).

2. Verfahren zur Herstellung der Verbindungen der allgemeinen Formel **A**, dadurch gekennzeichnet, daß man in an sich bekannter Weise aus Longifolen erhaltenes Isolongifolen zu Isolongifolan-3-on oxidiert und dies mit aliphatischen 1,2-Diolen unter Wasserabscheidung in apolaren Lösungsmitteln umsetzt.

3. Verwendung der Verbindungen der allgemeinen Formel **A** als Riechstoffe oder Bestandteile von Riechstoff-Mischungen bzw. Parfümölen zur Parfümierung von kosmetischen oder technischen Gebrauchsgütern.

## Claims

1. Cyclic Isolongifolanone ketals of the general formula A wherein the wavy lines mean α- and β- configuration and R,R′ mean radicals of hydrogen, methyl or ethyl,

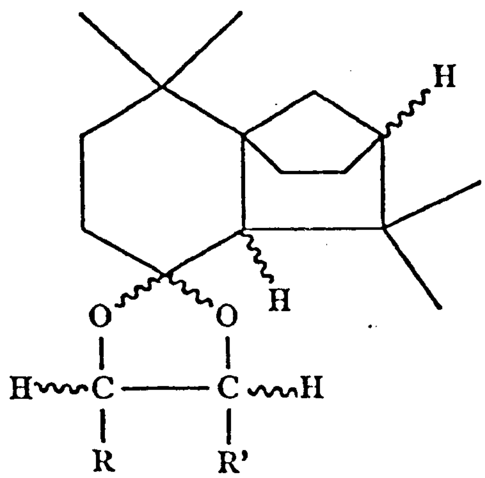

wherein the following combinations are preferred:
R=R′=H
R=H, R′=Me/R=Me, R′=H
R=H, R′=Et/R=Et, R′=H
R=R′ =Me (α,β)

2. Process for the production of compounds of the general formula A, characterised in that in a method known per se Isolongifolene obtained from Longifolene is oxidised to Isolongifolane-3-one and this is reacted with aliphatic 1,2-diols in apolar solvents with separation of water.

3. Use of compounds of the general formula A as odorants or components of mixtures of odorants or perfume oils for the perfuming of cosmetic or technical consumer goods.

**Revendications**

1. Acétals cycliques de l'isolongifolanone de formule générale A, les lignes ondulées signifiant une configuration α et β, et R, R′ signifiant respectivement des groupes hydrogène, méthyle ou éthyle,

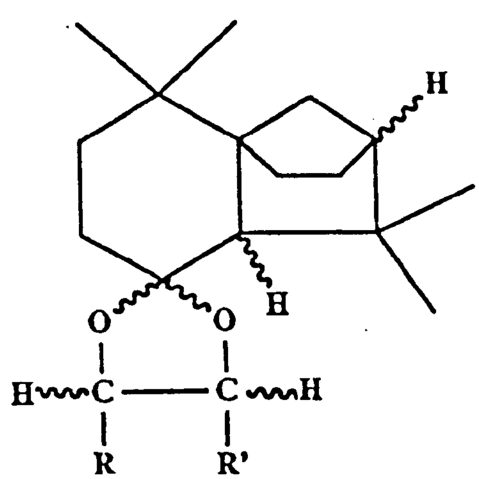

A

où on préfère les combinaisons suivantes:
R = R′ = H

R = H, R′ = Me/R = Me, R′ = H
R = H, R′ = Et/R = Et, R′ = H
R = R′ = Me ($\alpha$,$\beta$).

2. Procédé de fabrication de composés de formule A, caractérisé en ce que, d'une manière connue en soi, on oxyde l'isolongifolène obtenu à partir de longifolène en isolongifolan-3-one et on fait réagir celui-ci avec des 1,2-diolènes par élimination d'eau dans des milieux de solutions apolaires.

3. Utilisation des composés de formule générale A comme matière odorante ou comme constituant de mélanges de matières odorantes ou essences de parfum pour parfumer de produits utilitaires cosmétiques ou techniques.

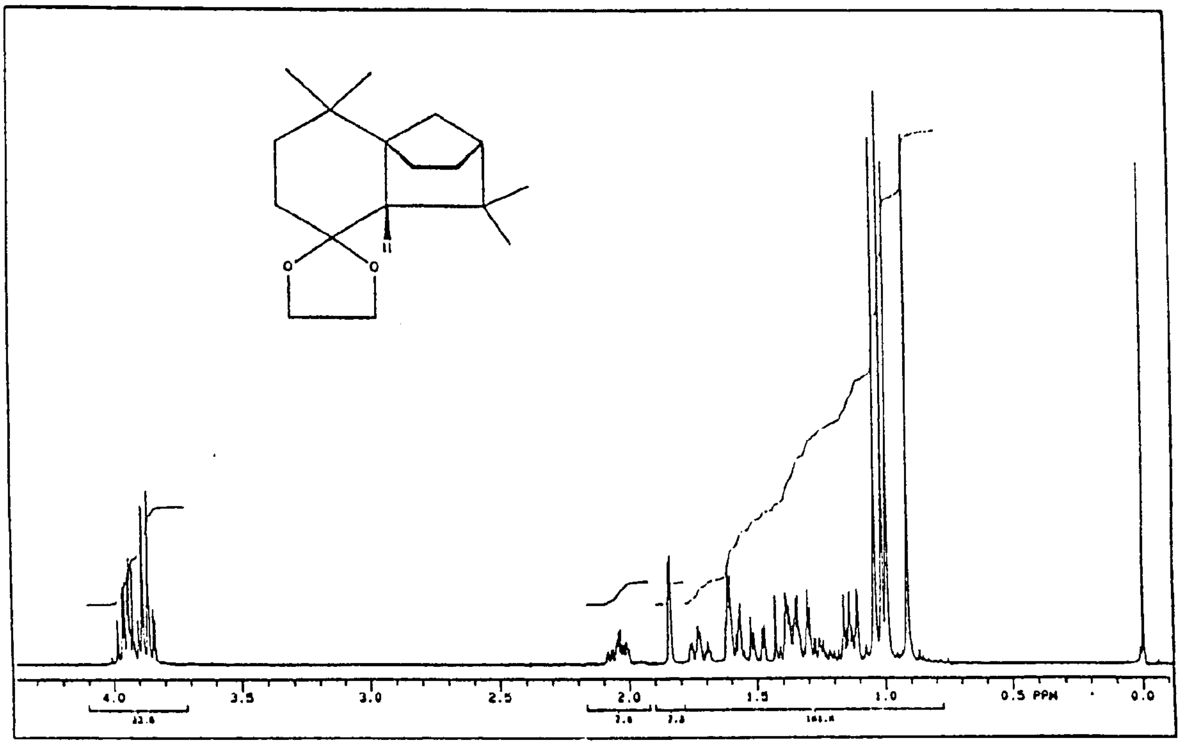


Abb. 1: $^{1}$H-NMR-Spektrum (300 MHz, $CDCl_3$) von **5a**

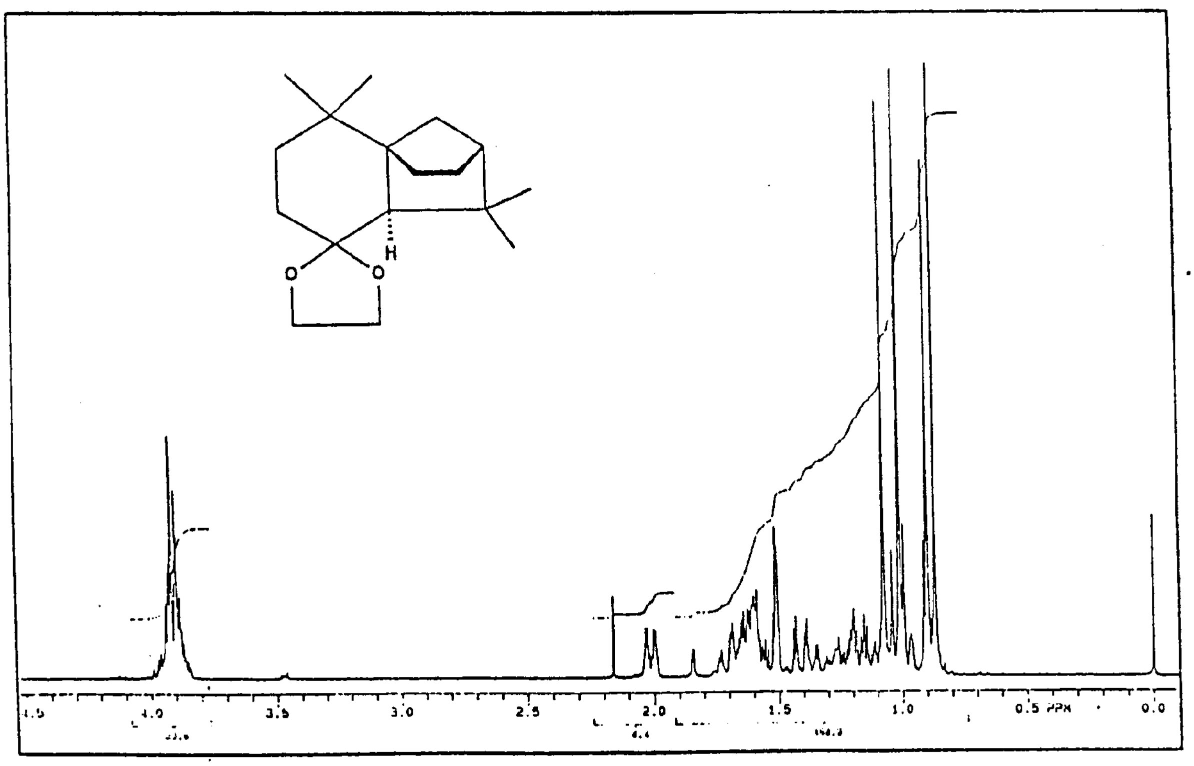


Abb. 2: $^{1}$H-NMR-Spektrum (300 MHz, $CDCl_3$) von **5b**